(19) **Europäisches Patentamt / European Patent Office / Office européen des brevets**

(11) **EP 3 467 118 B1**

(12) **EUROPEAN PATENT SPECIFICATION**

(45) Date of publication and mention
of the grant of the patent:
**02.12.2020 Bulletin 2020/49**

(51) Int Cl.:
***G01N 33/50*** *(2006.01)*

(21) Application number: **17805927.5**

(22) Date of filing: **29.05.2017**

(86) International application number:
**PCT/ES2017/070365**

(87) International publication number:
**WO 2017/207845 (07.12.2017 Gazette 2017/49)**

(54) **METHOD FOR EVALUATING THE CYTOTOXICITY OF CHEMICALS**

VERFAHREN ZUR BEURTEILUNG DER ZYTOTOXIZITÄT VON CHEMIKALIEN

PROCÉDÉ POUR ÉVALUER LA CYTOTOXICITÉ DE SUBSTANCES CHIMIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.05.2016 ES 201630708**

(43) Date of publication of application:
**10.04.2019 Bulletin 2019/15**

(73) Proprietor: **Universidad De Valladolid
47002 Valladolid (ES)**

(72) Inventors:
- **SRIVASTAVA, Girish Kumar**
  47002 Valladolid (ES)
- **ALONSO ALONSO, Ma Luz**
  47002 Valladolid (ES)
- **FERNÁNDEZ BUENO, Iván**
  47002 Valladolid (ES)
- **GARCÍA GUTIÉRREZ, Ma Teresa**
  47002 Valladolid (ES)
- **COCO MARTÍN, Rosa Ma**
  47002 Valladolid (ES)
- **PASTOR JIMENO, José Carlos**
  47002 Valladolid (ES)

(74) Representative: **Pons
Glorieta Ruben Dario 4
28010 Madrid (ES)**

(56) References cited:
- YANG A et al.: "Subacute cytotoxicity testing with cultured human lung cells", Toxicology In Vitro February, vol. 16, no. 1, 31 January 2002 (2002-01-31), pages 33-39, XP055443974, ISSN: 0887-2333
- BAKAND S et al.: "A novel in vitro exposure technique for toxicity testing of selected volatile organic compounds", JEM Journal of Environmental Monitoring JAN 2006, vol. 8, no. 1, 31 December 2005 (2005-12-31), pages 100-105, XP055443977, ISSN: 1464-0325
- BONSI PAOLA ET AL.: 'Two in vitro models for gas-phase exposure to volatile compounds' ATLA ALTERNATIVES TO LABORATORY ANIMALS vol. 30, no. 2, 28 February 2002, pages 241 - 247, XP055584510 ISSN: 0261-1929
- WILSON SAMANTHA L et al.: "An overview of current techniques for ocular toxicity testing", Toxicology, vol. 327, 2 January 2015 (2015-01-02), pages 32-46, XP055562651, ISSN: 0300-483X
- BAKAND SHAHNAZ et al.: "Troubleshooting methods for toxicity testing of airborne chemicals in vitro", Journal of Pharmacological and Toxicological Methods, vol. 61, no. 2, 28 February 2010 (2010-02-28), pages 76-85, XP055443980, ISSN: 1056-8719

## Description

The present invention falls within the field of healthcare and cytotoxicology, more specifically within the methods for evaluating the biosafety and biocompatibility of chemical products, particularly, of those containing highly volatile compounds, which are preferably used in clinical practice due to being considered biosafety products.

## STATE OF THE ART

Chemical healthcare products are widely used in clinical practice. These products are in direct, or indirect, contact with the human body and react with the environment and with the parts of the body where they are applied. Although they may have adequate mechanical, physical and chemical properties for certain medical applications, they also need to demonstrate their good biocompatibility and, therefore, demonstrate that they can be safely used in clinical practice (Directive 93/42/EEC of the Council, of 14 June 1993, relating to healthcare products), since during their use they may come into direct, or indirect, contact with human body cells and tissues.

Thus, the design of a strategy for evaluating the biocompatibility and safety of the application of a healthcare product is always a topic of interest that concerns the certification authorities of any country. International healthcare product standards (ISO 10993) provide a series of guidelines in this respect. Following these standards, healthcare products are subject to a series of rigorous biocompatibility tests, which include, among others, a cytotoxicity, sensitivity, intradermal irradiation and acute systemic toxicity analyses.

Cytotoxicity studies are a primary indicator of biocompatibility and, therefore, of healthcare product safety level (Li, W., Zhou, J. & Xu, Y. 2015, Biomedical Reports, vol. 3, no. 5, pp. 617-620). The results of this first study determine the future of the healthcare product: whether more in-depth research is required for it, whether modifications must be made, or whether it will be discarded in the initial manufacturing phase, prior to initiating studies with laboratory animals (Fricker, S. 1994, Toxicology in Vitro, vol. 8, no. 4, pp. 879-881). A good cytotoxicity assay is that which follows standard protocols, is highly sensitive and quickly produces quantitative and comparable data to be evaluated. Current advances in science and technology provide various types of assays for performing cytotoxicity studies, many of which are described in the aforementioned ISO standards. Following ISO standard recommendations, biocompatibility can be tested worldwide and, therefore, healthcare product safety level.

ISO 10993-5 standard recommends three types of cytotoxicity assays: the use of a sample extract, direct contact and indirect contact. To date, the extract dilution and indirect contact method have been the most widely accepted, since they can be applied to a wide variety of chemical products for use in healthcare and detect extractable and leachable toxins. Extraction conditions (time and temperature) depend on the physico-chemical characteristics of the healthcare product and of the extraction carrier. On the contrary, the direct contact method makes it possible to detect weaker levels of cytotoxicity and of non-extractable or leachable compounds, due to the high sensitivity thereof.

Mammal cell cultures are common in *in vitro* cytotoxicity studies; more specifically, established cell lines obtained from renowned repositories are used. ISO standards recommend cell lines CCL 1 (NCTC clone 929; mouse fibroblasts), CCL 163 (Balb/3T3 clone A31; mouse fibroblasts), CCL 171 (MRC-5; human fibroblasts), CCL 75 (WI-38; human fibroblasts), CCL 81 (Vero; non-human primate epithelial cells and CCL 10 [BHK-21 (C-13); hamster fibroblasts] and V-79 379A (hamster fibroblasts) of the American Type Culture Collection (ISO 10993-5:2009). However, the primary cell and tissue cultures obtained directly from living tissues are also recommended for performing analyses in which a specific sensitivity is required, provided that the reproducibility and accuracy of the cell and tissue response compared to the samples studied can be demonstrated. The choice of the cell and tissue cultures, the methodology followed to prepare the study samples, the type of cytotoxicity assay and analysis methods may vary in accordance with the standards of the certification authorities of the different countries.

Some manufactured polymers, such as for example perfluoro-n-octane, perfluorodecalin or sodium hyaluronate (viscoelastic solution), inter alia, are used as healthcare products. Perfluoro-n-octane, also known as octadecafluorooctane, is a liquid fluorocarbon, a perfluorinated derivative of the hydrocarbon octane. Its molecular formula is $C_8F_{18}$. It has a molar mass of 438.06 g/mol, a melting point of -13°F (-25°C) and a density of 1.77 g/cm$^3$. This healthcare product is very popular and widely accepted as an intraoperative mechanical tool in vitreoretinal surgery in patients with retinal detachment, penetrating eye trauma, giant retinal tear(s), proliferative vitreoretinopathy (PVR) or complications of cataract surgery. Perfluoro-n-carbon acts as a manipulator of the retina in vitreoretinal surgery or is used, for example, to refloat dislocated lenses into the vitreous cavity. Several healthcare companies market this product under different names such as Perfluoron®, Okta-line™ Arcotane (ARCADOPHTA®), Ala®Octa, F-Octane (FLUORON®) and Bio Octane™, inter alia.

The adequate synthesis and purification of the liquid perfluoro-n-octanes are always core issues for companies that manufacture and market them. Different techniques have been used, such as electrochemical fluoration, for the synthesis of raw materials and distillation and filtration for their purification. The biocompatibility of the raw materials and their safety are evaluated before bottling them in 5 or 7-millimeter syringes and vials and marketing them for ophthalmic use. Cytotoxicity studies are carried out in accordance with the guidelines of the ISO standard (UNE-EN ISO 10993-5:2009 Biological evaluation of healthcare products, Part 5: *In vitro* cytotoxicity assays and UNE-EN ISO 10993-12:2007 Biological evaluation of healthcare products, Part 12: Preparation of samples and reference materials). Assays performed using the extract dilution method by indirect contact can be applied in most cases.

The use of the NCTC 929 clone cell line [L cell, L-929, mouse subcutaneous connective tissue, derived from the L Strain] (ATCC® CCL-1™) is common in the performance of said assays. Standard protocols for evaluating cell proliferation are used to determine the cytotoxicity of liquid perfluoro-n-octane study samples. The study samples are extracted by stirring with a culture medium under certain time and temperature conditions, are observed to detect anomalies (clarity, color, absence of foreign matter) and are finally exposed to a cell culture of the L929 line. Furthermore, in the case of the agarose diffusion method, the study samples or extracts thereof are placed on top of an agarose mixture and then diffused towards the cell culture. In these assays, it is considered that the cytotoxic components of the perfluoro-n-octane, the healthcare product, would become separated in the extract or would be filtered through the agarose.

However, the recent cases of blindness after vitreoretinal surgery have alerted the certification/healthcare authorities in Europe, Asia and South America, which notified the healthcare authorities of the Autonomous Regions and the general public in relation to healthcare products containing perfluoro-n-octane (Spanish Agency for Medicine and Health Products. Web. Informative Note Ref. PS, 19/2015. Information on incidents related to the product ALA OCTA (perfluorooctane), used in retinal surgery; Swiss Agency for Therapeutic Products. Recalls and other field safety corrective actions (FSCA)-Archive, 21/12/2915; Chilean National Institute of Public Health (ISP) 27/08/2013. Withdrawal of the liquid perfluorooctane medical device manufactured by Meran Tip, Turkey and distributed by Falc Chile Ltda.; Department of Health, The Government of Hong Kong Special Administrative Region 17/12/2015. Medical Device Safety Alert: Arcadophta SARL Arcotane 5 ml). These alerts arise from research on healthcare products containing perfluoro-n-octane, particularly focused on the search for failures occurring in the current methods used to detect cytotoxicity in marketed batches of perfluoro-n-octane.

As mentioned earlier, perfluoro-n-octane is an example of a high-molecular-weight, volatile and non-water-soluble substance. Being non-water-soluble makes it necessary to use another, unknown, more adequate extraction carrier, wherein the toxic components released are soluble. The preparation of the samples of the compound under study, by means of stirring with a culture medium under certain conditions, cannot confirm the presence of non-water-soluble toxic components in the products. The method in which the extract of this sample is used would fail to determine cytotoxicity for this reason. Furthermore, its volatile nature makes it difficult to regulate the time of exposure of the compound samples under study to the cell and tissue cultures, and more so in a highly volatile product such as perfluoro-n-octane. The assay by indirect contact would also fail for this reason. Additionally, the cellular response would be different to different stimuli. For example, in a study published in 2015 (Zhongguo Yi Liao Qi Xie Za Zhi., 2015 Mar;39(3):212-5, In Vitro Cytotoxicity Study of Nickel Ion), cell lines L929, h9c2(2-1), 293[HEK-293], hFOB1.19, THLE-3, H9 and IM-9 were tested against healthcare products containing nickel, and the results confirmed that the cells of line L929 were the cells that best tolerated exposure to nickel (less sensitive). Therefore, the cellular response obtained in this type of experiment to determine cytotoxicity is also dependent on the cell type used.

Finally, it has been disclosed methods for evaluating the cytotoxicity of volatile chemical compounds on cells or tissues involving depositing the compound on the cells, overlaying this with a layer of mineral oil, incubating and evaluating the effect of the compound on the culture (Yang A., etal., 2002, Toxicology In Vitro, vol. 16:1, 33-39).

Therefore, taking into account the clinical problems caused by the toxicity of the substances such as that described previously and the difficulties for the toxicological analysis of the non-water-soluble volatile compounds, due to the impossibility of performing an adequate cytotoxicity assay, a new strategy must be designed to sensitively and reliably evaluate the biocompatibility, and therefore safety, of chemical healthcare products that may contain volatile or non-water-insoluble substances or non-extractable or leachable compounds prior to carrying out *in vivo* studies and its use in clinical practice.

## DESCRIPTION OF THE INVENTION

The present invention provides a method for evaluating the cytotoxicity of the chemical substances used in the preparation of healthcare products, specifically water-immiscible volatiles, which are the most complicated to analyze. The method makes it possible to evaluate cytotoxicities in a reliable, reproducible, controlled and highly sensitive manner.

When the toxic effects of highly volatile substances are evaluated *in vitro* through a study involving direct contact with the cell cultures it is difficult to control real exposure times, which would make it impossible to carry out an adequate, reliable and reproducible toxicological study. Additionally, when said substances are water-immiscible, the diffusion of the toxic compounds present therein towards the cell or tissue culture medium is impeded. The method proposed by the present invention provides a solution to all these problems, since it includes the use of a non-volatile substance, preferably an aqueous medium, disposed on the volatile compound under study, thereby preventing it from evaporating and, therefore, making it possible to control the time of exposure of the cells or tissue to the volatile chemical products studied. This makes it possible to reliably and reproducibly determine the biocompatibility and biosafety of these volatile chemical products prior to carrying out *in vivo* and clinical practice studies, i.e. prior to coming into contact with the patient. Additionally, this method provides direct contact for a controlled exposure time between the cell and/or tissue culture and the volatile compound, making it possible to detect cytotoxicity levels with high sensitivity, including the detection of the cytotoxicity associated with compounds with mild toxicity. The invention makes it possible, using the described method, to evaluate the biocompatibility and safety, and therefore toxicity, of a large number of healthcare products that come into contact with patients during clinical practice, providing useful, specific and tangible results.

The advantages associated with the method proposed by the present invention are, therefore, as follows:

- It enables direct contact between the volatile compound and the cells and/or tissues, which implies that there is no interference with any other factor, as in the case of assays by indirect contact or in extract dilution methods, wherein other factors are involved, such as extraction carriers, the agar layer over the cell layer, etc., that may be affecting cell proliferation or tissue structure in the culture and, therefore, the assay results.
- It prevents the volatile substance under study from evaporating during the assay, thereby making it possible to regulate and control the time of exposure thereof to cell or tissue cultures.
- It makes it possible to clearly confirm what compound is in direct contact with the cells and tissues since, due to the insoluble nature of the volatile compound and to its difference in molecular weight with the aqueous medium with which it is in contact, the different layers of the system can be easily observed.
- It makes it possible to collect the substances that have come into contact with the cells and tissues after the desired exposure time for subsequent studies.

Thus, this method facilitates testing of volatile chemical products, preferably healthcare products, generating sufficient guarantees to evaluate the biocompatibility and safety thereof.

Therefore, one aspect of the invention relates to a method for evaluating the cytotoxicity of volatile chemical compounds, which comprises:

a. depositing a volatile chemical compound to be studied on an *in vitro* cell or tissue culture,
b. depositing a non-volatile aqueous medium on the compound deposited in step (a),
c. incubating the cell or tissue culture obtained after step (b), and
d. evaluating the effect of the presence of the compound deposited on the culture;

wherein the volatile chemical compound deposited is water-insoluble and has a higher molecular weight than that of the non-volatile aqueous medium of step (b); and wherein the amount of compound deposited in step (a) covers the cultured cell or tissue layer and the amount of non-volatile aqueous medium deposited in step (b) covers the compound layer deposited in step (a).

Hereinafter, this method shall be referred to as the "method of the invention".

The term "volatile compounds", "VOCs" or "COVs" refers to any organic compound (containing carbon) that easily evaporates into the atmosphere at room temperature. They are chemical substances containing carbon and one or more of the following elements, but not limited to: hydrogen, halogens, oxygen, sulfur, phosphorus, silicon or nitrogen, except carbon oxides and carbonates and inorganic bicarbonates. Furthermore, the volatile compounds are easily transformed into vapors or gases and can also pose a health problem since they may have, for example, an irritating effect on the eyes and respiratory tract, possibly triggering asthmatic reactions. Its main features are volatility, liposolubility, flammability and sometimes toxicity. Some examples of these compounds are, but not limited to, perfluoro-n-octane, perfluorodecalin, sodium hyaluronate, toluene, formaldehyde, butane, propane, xylene, butyl alcohol, methyl ethyl ketone, acetone, ethylene glycol, isoprene, pinene, limonene, benzene, nitrobenzene, chlorobenzene, perchloroethylene (or tetrachloroethylene), trichloroethylene, hydroxypropylmethylcellulose, silicone oil, etc., which can be tested using the method of the invention.

The volatile chemical compound to which the present invention relates is a water-insoluble and high-molecular-weight compound. The term "high molecular weight" refers to the fact that the volatile compound has a higher molecular weight than that of the non-volatile aqueous medium of step (b). That is, the volatile compound to which the present invention relates is unable to float in an aqueous medium, preferably in the non-volatile aqueous medium used in the method of the present invention that is deposited thereon. Thus, the volatile compound under study remains underneath the non-volatile aqueous medium deposited in step (b) during the method of the invention, i.e. it does not float thereon. And in turn, said non-volatile aqueous medium will prevent the evaporation of the volatile compound situated underneath. This enables direct and permanent contact (during the desired exposure time) of the volatile compound with the cell or tissue culture. The method of the present invention therefore, allows the cultured cells or tissues to be in direct contact with the volatile compound samples for a specific exposure time. If the volatile compound sample is toxic or contains leachable toxic components, it will directly affect said cultures and its effect may be subsequently evaluated.

The term "cell culture" refers to a eukaryotic or prokaryotic cell culture, preferably eukaryotic, more preferably mammal (for example human, feline, canid, bovine, rodent or pig), even more preferably human. The three types of cells: ectodermal, mesodermal and endodermal cell derivatives, can be used in the method of the invention. The preferred cell lines for the cell culture of step (a) are those recommended by ISO standards (ISO 10993-5:2009), for example, but not limited to, cell lines CCL 1 (NCTC clone L929; mouse fibroblasts), CCL 163 (Balb/3T3 clone A31; mouse fibroblasts), CCL 171 (MRC-5; human fibroblasts), CCL 75 (WI-38; human fibroblasts), CCL 81 (Vero; non-human primate epithelial cells) or CCL 10 [BHK-21 (C-13); hamster fibroblasts] and V-79 379A (hamster fibroblasts) of the American Type Culture Collection. However, the primary cell and tissue cultures obtained directly from living tissues are also recommended for carrying out the method of the invention.

The "tissue culture" includes from the culture of organs to the culture of isolated tissues. All type of tissues: ectodermal, mesodermal and endodermal tissue derivatives, may be used in the method of the invention, for example, but not limited to, epithelial tissue, eye tissue, muscle tissue, nerve tissue, bone tissue, conjunctive tissue, connective tissue, etc.

Preferably, the cells and tissues used in the cell or tissue culture of step (a) are cells and tissues from the part of the body where the healthcare product will be applied or with which said sanitary product will be in contact. The evaluation of healthcare products over the types of cells and tissues where they are usually applied in clinical practice provides greater assurance of their biocompatibility and, therefore, of their safety.

In a preferred embodiment of the method of the invention, the cell or tissue culture comprises neuroretina explants and/or retinal pigment epithelium (RPE) cells. In a more preferred embodiment, the neuroretina explants and retinal pigment epithelium are of human origin. In an even more preferred embodiment, the RPE cells are cells of the cell line ARPE-19 (ATCC CRL-2302), which is a cell line spontaneously generated from human RPE cells. These cells form a stable monolayer that exhibits morphological and functional polarity. ARPE-19 cells express specific RPE markers, such as CRALBP and RPE-65 and exhibit morphological polarization when sown on Transwell-COL filters covered with laminin and in a culture medium with low serum concentration. These cells form strong bonds in monolayers with transepithelial resistance. Also, other sources of RPE cells and other cultured retinal cells, such as for example, but not limited to, photoreceptors, ganglions, Müller, etc., and not only of modified human origin, but rather fresh and of other animals, can also be used in the embodiment of the method of the invention.

Preferably, the neuroretina explants to which the present invention relates are explants obtained under laboratory conditions from living tissue.

The term "neuroretina explants" refers to a neuroretina tissue, i.e. a tissue obtained from the innermost layer, in relation to the position of the pigment epithelium, of the two parts of the retina. The neuroretina is the layer of the retina that gives rise to the nerve impulse triggered by the photons. It consists in turn of nine layers which, from the outside towards the inside, are as follows: layer of cones and rods, external limiting membrane, outer nuclear layer, outer plexiform layer, inner nuclear, inner plexiform layer, ganglion cell layer, nerve fiber layer and inner limiting membrane. The neuroretina explants can be cultured in the presence of the culture media and conditions known in the technical field for culturing eye tissue explants. Thus, the culture medium may comprise, for example, but not limited to, fetal bovine serum (FBS) or fetal human serum, antibiotics, antifungals, growth factors, etc. The base medium that can be used in the culture medium may be any of those known in the state of the art for *in vitro* tissue culture, such as for example, but not limited to, Neurobasal A medium.

The "retinal pigment epithelium" or "RPE" is the pigmented cell layer located on the outer part of the retina that interacts closely with the photoreceptor cells (cones and rods) in the maintenance of the visual function. It is firmly anchored to the underlying choroid by Bruch's membrane. The retinal pigment epithelium is composed of a layer of hexagonal cells that are densely packed with pigment granules. Seen in a cross-sectional view, each cell consists of a non-pigmented outer part in which a large, oval nucleus is located and a pigmented inner part that extends a series of straight filiform processes between the rods. It serves as a limiting factor of the transport that maintains the environment of the retina, supplying small molecules such as amino acids, ascorbic acid and D-glucose, while representing a narrow barrier for the substances transported by the choroid blood supply. The retinal pigment epithelium is responsible for photoreceptor cell outer segment phagocytosis and for photopigment regeneration.

"Retinal pigment epithelium cells" or "RPE cells" are understood to be any cell type present in said epithelium, preferably epithelial cells. RPE cells can be cultured in the presence of culture media and conditions known in the technical field of epithelial cell culture. Thus, the culture medium may comprise, for example, but not limited to, fetal bovine serum (FBS) or fetal human serum, antibiotics, antifungals, growth factors, etc. The base medium that can be used in the culture medium may be any of those known in the state of the art for *in vitro* cell culture, such as for example, but not limited to, "Eagle" basal medium, CRCM-30, CMRL-1066, "Dulbecco's Modified Eagle's Medium" (DMEM), "Eagle's Minimum Essential Medium" (EMEM), "Fischer's Medium", "Glasgow Minimum Essential Medium", Ham's F-10, Ham's F-12 (F12), "High Cell Density Medium", "Iscove's Modified Dulbecco's Medium", Leibovitz's L-15, McCoy's 5A, 199 medium, "Minimum Essential Medium Eagle", "Alpha Minimum Essential Medium", CnT20, NCTC 109, NCTC 135, RPMI-1640, "William's Medium E", Waymouth's MB 75211, Waymouth's MB 70511, "Keratinocyte serum-free medium" (KSFM), or any combination thereof. Preferably, the culture medium comprises a DMEM/F12 base medium, supplemented or not with FBS and in the presence of antibiotics and antifungals. Additionally, the culture conditions may be, for example, but not limited to, standard culture conditions, i.e. in the presence of between 5% and 10% of $CO_2$, between 36ºC and 38ºC and renewing the medium every 48 to 72 hours. Preferably, said culture is carried out as described in the following examples of the present invention.

The *in vitro* cell or tissue culture in the method of the present invention is carried out, preferably, as indicated by ISO standard guidelines (UNE-EN ISO 10993), more preferably following the protocol for preparing culture plates.

In the method of the invention, a product, namely a non-volatile aqueous medium, is deposited, on the volatile compound under study in order to prevent the latter from evaporating and disappearing before the end of the exposure time.

The term "non-volatile aqueous medium" refers to a liquid medium that cannot easily evaporate into the atmosphere at room temperature, which in turn prevents the evaporation of the volatile compound under study once deposited thereon. The volatile compound under study is insoluble in said medium. This medium shall preferably be water or any aqueous medium wherein the volatile compound under study does not dissolve. The non-volatile aqueous medium used in the present invention does not contain elements or components that could compromise cell or tissue viability or growth or that can affect the physico-chemical properties of the volatile compound under study. That is, the components comprised in the non-volatile aqueous medium used in the present invention shall be harmless both to the cell or tissue culture and to the volatile compound under study. Said aqueous medium is, preferably, alkaline. In another preferred embodiment of the method of the invention, the non-volatile aqueous medium of step (b) is a culture medium having an identical or similar composition to that used in the cell or tissue culture of step (a) of the method.

The amount of the volatile compound under study and of the non-volatile aqueous medium depend on the surface of the medium or on the volume of the receptacle wherein the cells or tissues are being cultivated, and it should also be taken into account that they must not generate weight/pressure such as to crush the cultured cells or tissues. To avoid this problem, negative controls such as those described below shall, preferably, be used in the method of the invention.

In another preferred embodiment of the method of the invention, the culture of step (a) is a cell culture, the amount of the volatile compound deposited in step (a) is 80 $\mu$l or any other amount that can cover the layer of cultured cells and the amount of non-volatile aqueous medium deposited in step (b) is 100 $\mu$l or any other amount that can cover the compound layer deposited in step (a).

In another preferred embodiment of the method of the invention, the culture of step (a) is a tissue culture, the amount of the volatile compound deposited in step (a) is 500 $\mu$l or any other amount that can cover the cultured tissue layer and the amount of non-volatile aqueous medium deposited in step (b) is 500 $\mu$l or any other amount that can cover the compound layer deposited in step (a).

The incubation of the cell or tissue culture of step (b) in the presence of the volatile compound and of the non-volatile aqueous medium, as described in step (c) of the method of the present invention, takes place during a certain exposure time (desired and preferably based on the time during which the compound will be in contact with the cells and tissues in the organism, preferably human, during real clinical practice) and preferably in the presence of the appropriate conditions for enabling cell or tissue growth or proliferation. Said culture conditions and time will depend on the type of cell or tissue chosen for the culture. The persons skilled in the art will recognize said incubation conditions and time applicable in each case, although the incubation of step (c) is preferably carried out for at least 30 minutes.

In a particular embodiment of the method of the invention, for the cell culture of step (a) the cells are sown on a multi-well plate and the cells of the second column on the right and the second column on the left are used as controls to evaluate the quality of the analysis. In accordance with the guidelines of the ISO standard (UNE-EN ISO 10993-5:2009 Biological evaluation of healthcare products, Part 5: *In vitro* cytotoxicity assays; Appendix C: MTT toxicity assay) the average optical density value ($OD_{570}$) of the control groups of the second column on the right and on the left must be $\geq$ 0.2 and the difference between the average $OD_{570}$ value of the controls of the second column on the right and on the left must not be greater than 15% of the total average of both control columns.

Thus, in another preferred embodiment, the method of the invention is carried out on a multi-well plate wherein at least two columns of the plate comprise control cell or tissue cultures. In a more preferred embodiment, the average optical density value ($OD_{570}$) of the control cell cultures of the two columns is at least 0.2. In an even more preferred embodiment, the difference between the average $OD_{570}$ value of the control cell cultures of the two columns is equal to or less than 15% of the total average of both columns. In the case of the control tissue cultures, fresh tissue from the day of isolation and preparation of the experiments is preferably applied to evaluate the quality of the analysis.

Said control cultures comprise cells of the same cell type and same source as the cells cultured in step (a) of the method of the present invention, or tissue of the same type and same source as the as the tissues cultured in step (a) of the method of the present invention. Said control culture cells and tissue are incubated under the same conditions of time, temperature, pH, light/darkness cycles, humidity, $CO_2$ concentration, culture medium composition, etc., as the culture under study of step (a), but said control cultures are not exposed to the volatile compound added in step (a). The control cultures will also be processed in the same manner as the cultures of step (a) after the incubation of step (c).

In a more preferred embodiment, the control cultures comprise cells or tissues not exposed to any compound (blank), cells or tissues exposed to a non-toxic compound with a molecular weight identical to that of the deposited volatile compound under study (negative control) or cells or tissues exposed to a control toxic compound (positive control). The negative control comprises a non-toxic compound with a molecular weight identical to that of the volatile compound under study in order to help determine that the effects on the cells or tissues arise from the volatile compound under study and not from damage caused by crushing due to the weight/pressure exerted by the deposited substances.

ISO standard (UNE-EN ISO 10993-12:2007 Biological evaluation of healthcare products, Part 12: Preparation of reference samples and materials) mentions that those materials whose effects have been proven to be reproducible can be considered positive controls (reproducible cytotoxic effect) and negative controls (reproducible non-cytotoxic effect). In this way, the state of the cell or tissue culture can be determined, i.e. the response of the cells or tissues to the stimulus, to carry out the evaluation by direct contact. In an even more preferred embodiment, the control toxic compound is selected from the list, mentioned in the ISO standard, consisting of: PVC-org. Sn, SPU-ZDEC, SPU-ZBEC, natural latex and polyurethane or phenol. However, any other chemical substance that gives rise to reproducible results can be applied. In a particular embodiment, the control toxic compound is phenol.

Prior to step (a) of the method, the cells and tissues under study, in addition to the controls, can be cultured on a multi-well plate, preferably for at least 7 days or until the average optical density value ($OD_{570}$) is obtained from the cultures of at least 0.2 in the case of the cells, and the culture medium is subsequently removed and are incubated again with incomplete medium (without serum but with antibiotics and antifungals) under standard culture conditions for preferably 24 hours to synchronize the cell cycle. In the case of tissues, they are isolated and fresh tissue is preferably prepared on the same day as the start of the experiments. Subsequent to this culture phase, the volatile compound is deposited on the culture of each well (except the controls) so it is in direct contact with the cells or tissues, as described in step (a) of the method of the invention. Next, the non-volatile aqueous medium (preferably culture medium) is deposited on the samples of the deposited volatile compound and the samples are incubated for, preferably, at least 30 minutes. This 30-minute exposure period may vary, adjusting the time during which the compound under study would be in contact with cells and tissues in the organism, preferably human, during real clinical practice.

The toxicity data of the studied samples are always comparable to the data of the cultures not exposed to any compound (blank) using, preferably, the following formula (in the case of cells) or Table 1 (in the case of tissues).

In the case of the cell culture,

$$Viab.\% = \frac{100 \times OD570e}{OD570b}$$

according to standard UNE-EN ISO 10993-5 "C.2.5 Data analysis", OD570e is the average optical density value measured in the assay samples and OD570b is the average optical density value measured in the blanks.

Thus, in an even more preferred embodiment, the cytotoxicity of the cell culture studied is compared with the cytotoxicity of the control cultures that comprise cells not exposed to any compound following the formula:

$$Viab.\% = \frac{100 * OD570e}{OD570b}$$

OD570e being the average optical density value in the cell culture studied and OD570b being the average optical density value in the control culture that comprises cells not exposed to any compound.

And in the case of the tissue culture,

subjective qualitative grading of the tissue/cell modifications in the neuroretina explants. Table configured from Spanish Standard UNE-EN ISO 10993-5:2009 "Table 1 - Qualitative morphological evaluation of the cytotoxicity of the extracts".

| Degree | Degeneration | Culture conditions |
|---|---|---|
| 0 | None | Absence of degenerative changes in the neuroretina explants |
| 1 | Light | Presence of light degenerative changes, such as: partial absence of outer photoreceptor segments and start of tissue vacuolization |

(continued)

| Degree | Degeneration | Culture conditions |
|---|---|---|
| 2 | Mild | Presence of mild degenerative changes, such as: fragmentation and loss of outer photoreceptor segments; photoreceptor inner segment edema; formation of degeneration rosettes; reduction of the number of nuclei in the nucleus layers; alteration of the plexiform layers; presence of pyknotic nuclei; and tissue vacuolization |
| 3 | Moderate | Presence of moderate degenerative changes, such as: absence of photoreceptor inner segments; alteration and reduction of the plexiform layers; loss of retinal parenchyma; disorganization of the retinal structure; and tissue vacuolization of cell nuclei and pyknosis in the present cells; and marked tissue vacuolization throughout the retinal parenchyma |
| 4 | Severe | Presence of severe degenerative changes, such as: total loss of the retinal structure; marked loss of cell nuclei; and marked loss of retinal parenchyma |

In order to "evaluate the effect caused by the presence of the volatile compound deposited on the culture", one or several parameters associated with the determination of the cell viability, growth, proliferation and/or survival or associated with the determination of the damage in the morphology of the cells or in the anatomical structure or morphology of the cultured tissue can be detected or quantified in said culture. The measurement of these parameters is made, preferably, following the standard cytotoxicity protocols described in the ISO standards (UNE-EN ISO 10993-5:2009 Biological evaluation of healthcare products, Part 5: *In vitro* cytotoxicity assays; UNE-EN ISO 10993-5:2009 "Table 1 - Qualitative morphological evaluation of extract cytotoxicity"). In the case of the cell cultures, a MTT cytotoxicity assay can be carried out, for example but not limited to, a MTT cytotoxicity assay following the guidelines of the ISO standards wherein cell growth is evaluated (UNE-EN ISO 10993-5:2009 Biological evaluation of healthcare products, Part 5: *In vitro* cytotoxicity assays; Appendix C: MTT toxicity assay). Additionally, the damage caused can be evaluated by verifying the morphology of the cultured cells by microscopy during cell growth time. A significant change in the appearance of the culture can be detected in comparison with the control culture. In the case of the tissue cultures, the samples can be, for example but not limited to, fixed, processed and stained with toluidine blue for the subsequent visual evaluation of tissue morphology under the microscope.

In a more preferred embodiment of the method of the invention, the cell proliferation and/or survival or morphology of the tissue is evaluated in step (d).

When, in step (d), significant changes are detected in cell proliferation and/or survival or in the morphology of the tissue with respect to the "control" culture not exposed to the volatile compound added in step (a) of the method of the invention, said volatile compound is classified as cytotoxic. When in step (d) no significant changes are detected in cell proliferation and/or survival or in the morphology of the tissue with respect to the "control" culture not exposed to the volatile compound added in step (a) of the method of the invention, said volatile compound is classified as non-cytotoxic.

In another preferred embodiment of the method of the invention, it comprises an optional additional step (c') after step (c) of incubation wherein the cultures are subject to washing, preferably with PBS or culture medium, to remove remaining volatile compound, fresh culture medium is added and incubated again for a certain time period, preferably for at least 24 hours in the case of cultured cells or for at least 72 hours in the case of cultured tissues, under standard culture conditions. Subsequent to this additional step (c'), the effect caused by the volatile compound is evaluated as described in step (d).

In another preferred embodiment of the method of the invention, it further comprises a step (e) wherein the volatile compound deposited in step (a), is recovered. The insolubility of the volatile compound in aqueous media facilitates the clear marking of the samples evaluated in said compound in contact with the cells. The volatile compound sample is insoluble in the culture medium, which helps to clearly and visibly mark the layer with the sample of the product under study. Once the volatile compound has been recovered and collected in step (e), it can be reused in new studies and analyses.

Volatile compounds are usually found in industrial sectors, such as, but not limited to, steel, plastic and rubber, footwear, graphic arts, paints, varnishes, lacquers, solvents, repellents, glues, dispersants, degreasers, cleaning products, flavorings, perfumery and aerosols, food, timber, cosmetics, fracking, and pharmaceutical industry, this being the most relevant with respect to the invention presented herein. Furthermore, it also includes, but not limited to, the products used as electrical, thermal or acoustic insulators and semi-finished plastic materials, in the form of sheets, plates or rods, as well as rubber, gutta-percha, gum, asbestos, mica and products made of these materials not comprised in other classes, products made from semi-finished plastic products, materials used to caulk, seal with tow and insulate non-metallic flexible tubes, etc., without ruling out the volatile products that are released as a consequence of burning fossil fuels, such as gasoline, coal or natural gas, or from the burning of wood. Thus, the volatile compounds evaluated in the method of the present invention may be any of those present in said industry sectors, or in any other sector wherein volatile compounds are also used or released.

In another preferred embodiment of the method of the invention, the volatile compound deposited in step (a) is a compound used in the preparation of medical devices or healthcare products.

The term "medical device" refers to any instrument used in the healthcare field or clinical practice, both in medicine and veterinary medicine, which is in direct or indirect contact with the patient. This includes any instrument, apparatus, device, biomedical equipment, reagent for diagnosis or supervision or other similar or related article, used alone or in combination, including their components, parts and accessories involved in the proper application thereof, intended by the manufacturer for use in humans or non-human animals, preferably in humans. This term includes the devices used in the treatment, relief, diagnosis, prognosis, prevention, supervision, follow-up or monitoring, research and/or surgery of the patient, including the devices intended for replacing, modifying or supporting the anatomical structure of a tissue or a physiological process or the devices used in control of conception. Thus, this term includes the prostheses or devices implantable in the human or animal body intended for treating a pathological condition or for tissue regeneration.

Some examples in the context of the present invention are, but not limited to:

- Chemical healthcare products for medical use: dietary substances for medical use, baby food; plasters, material for bandages; material for filling teeth and for dental molds; disinfectants; products for the destruction of harmful animals; fungicides, herbicides.
- Diagnostic or therapeutic apparatuses and instruments (surgical or medical, including those for dentistry and veterinary use), coated with chemical products that can come into contact with skin or mucosa, eyes and artificial teeth; orthopedic articles; and material for sutures, syringes, needles, gauzes, catheters, bandages, gloves, particularly those used in surgery, pacemakers, bone matrix implants, prostheses, contact lenses, etc.
- And any medical device or healthcare product containing chemical products that may be in contact with animal cells and tissue, preferably human.

In a more preferred embodiment, the medical devices to which the present invention relates are used in surgery, even more preferably in ophthalmic surgery.

The term "ophthalmic surgery" makes reference to any surgical technique carried out in any eye structure, such as, but not limited to, refractive surgery, vitreoretinal surgery, glaucoma surgery, cataract surgery, inter alia. Vitreoretinal surgery is an eye operation for treating diseases that affect the vitreous and retina. These types of diseases or trauma cause retinal detachment, i.e. retinal tear and accumulation of the fluid in the vitreous cavity under the retina. Thus, vitreoretinal surgery is applied, but not limited to, patients with retinal detachment, penetrating eye trauma, giant retinal tear(s), proliferative vitreoretinopathy (PVR) or complications of cataract surgery.

In another preferred embodiment of the method of the invention, the volatile compound is a manufactured polymer.

The term "manufactured polymer" refers to polymers formed from natural or synthetic polymers. Furthermore, polymers are macromolecules formed when smaller molecules called monomers bond together. Some of the natural polymers from which the manufactured polymers are generated are starch, cellulose or silk and some of the synthetic polymers from which the manufactured polymers are generated are nylon, polyethylene or backelite. Examples of manufactured polymers are, but not limited to, perfluoro-n-octane, perfluorodecalin or sodium hyaluronate (viscoelastic solution), inter alia, which are used as healthcare products.

In a more preferred embodiment, the volatile compound is perfluoro-n-octane.

The term "perfluoro-n-octane" or "octadecafluorooctane" refers to liquid fluorocarbon, a perfluorated derivative of the hydrocarbon octane, whose molecular formula is $C_8F_{18}$, with a molar mass of 438.06 g/mol, a melting point of -13°F (-25°C) and a density of 1.77 g/cm$^3$. This polymer is applied in ophthalmology, particularly in vitreoretinal surgery, acting as a retinal manipulator due both to its good physical properties and to its good adhesion to the retina. Therefore, this polymer comes into contact with the patient's eye cells and tissues during vitreoretinal surgery, making it necessary to evaluate their toxicity. The retinal pigment epithelium is one of the layers of the retina that is exposed to the perfluoro-n-octane during said surgery, in the event of neuroretinal breaks. Accordingly, human RPE cells are preferably used in the method of the invention, due to being considered the most appropriate cells for evaluating the cytotoxicity of this volatile chemical product. In vitreoretinal surgery, the neuroretina is also exposed to the perfluoro-n-octane, therefore neuroretinal explants are also appropriate for evaluating the cytotoxicity of this type of healthcare product using the method described herein. Several companies market this perfluoro-n-octane product under different names such as Perfluoron®, Okta-line™, Arcotane (ARCADOPHTA®), Ala®Octa, F-Octane (FLUORON®) and Bio Octane™, inter alia.

Throughout the description and claims, the word "comprises" and its variants do not intend to exclude other technical features, additives, components or steps. For persons skilled in the art, other objects, advantages and features of the invention may be derived both from the description and the practical use of the invention. The following examples and figures are provided by way of example and do not intend to limit the present invention.

## DESCRIPTION OF THE FIGURES

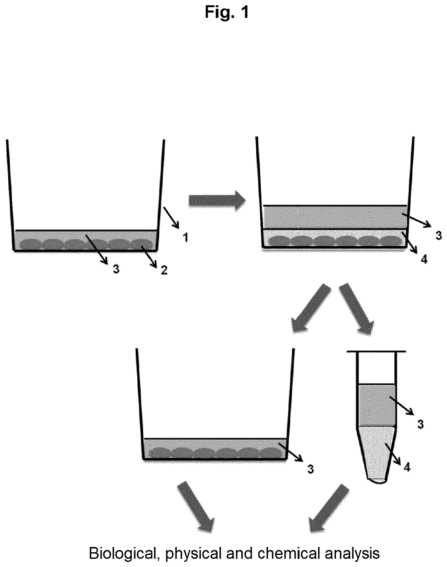

**Fig. 1. Design diagram of the method of the invention.** 1) Culture plate, 2) cells or tissues 3) culture medium, 4) assay sample.

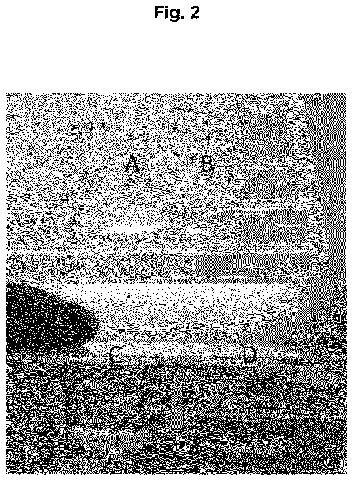

**Fig. 2. Culture plates showing the method of the invention designed for carrying out the evaluation by direct contact.** A) perfluoro-n-octane study sample in a 96-well plate well. B) culture medium on a perfluoro-n-octane study sample in a well of a 96-well plate. C) culture medium on a perfluoro-n-octane study sample in a well of a 12-well plate. D) culture medium in a well of a 12-well plate.

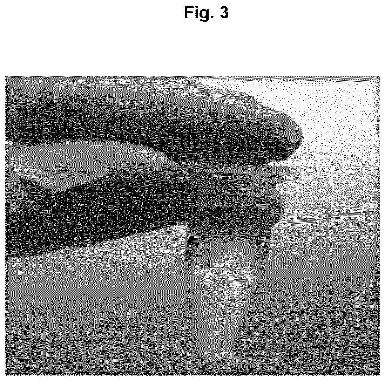

**Fig. 3. The volatile perfluoro-n-octane sample (bottom layer) was collected after the exposure time** on the cell culture or on the tissue section had elapsed.

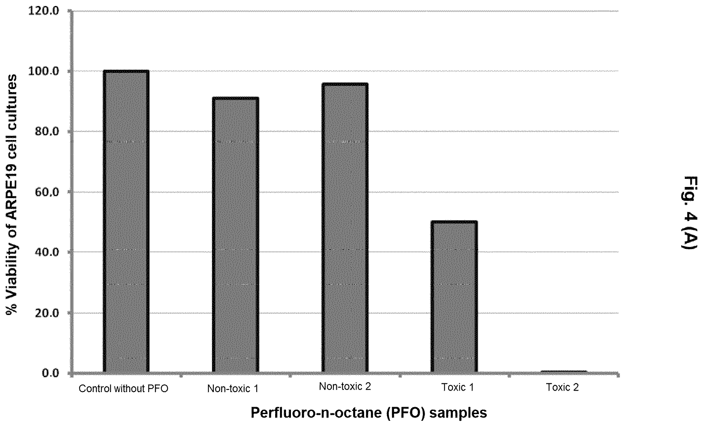

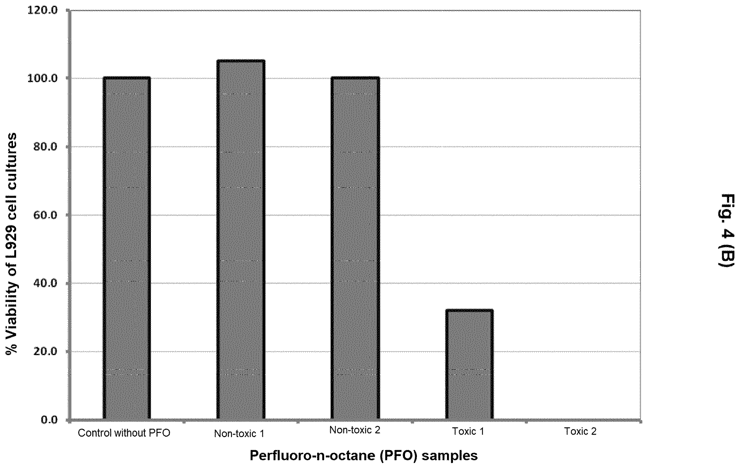

**Fig. 4. Proliferation of the ARPE-19 (A) and L929 (B) cell culture.** The cell cultures were in contact with toxic and non-toxic perfluoro-n-octane (PFO) samples for 30 minutes. They were incubated with culture medium for 24 hours under standard culture conditions of 37ºC and 5% of $CO_2$. Next, a MTT assay was performed following ISO standard guidelines, and the results obtained are shown in this graph. The y-axis represents the percentage of cell viability and the x-axis represents the different treatments.

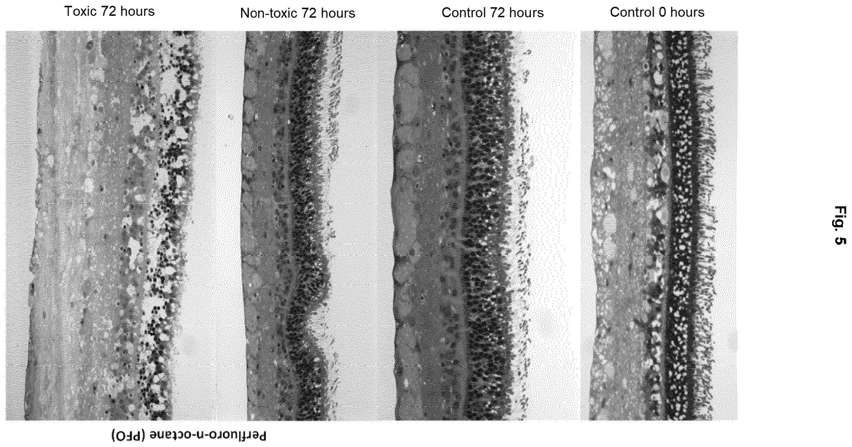

**Fig. 5. Morphology of the neuroretina explants.** The neuroretina explants were in contact with toxic and non-toxic perfluoro-n-octane samples for 30 minutes. They were incubated with culture medium for 72 hours under standard culture conditions of 37ºC and 5% of $CO_2$. Next, the morphology of each neuroretina explant was analyzed by staining with toluidine blue and the results are presented following ISO standard guidelines. It shows: the morphology of neuroretina explants at 0 time without incubating with culture medium, the morphology of neuroretina explants not exposed to perfluoro-n-octane and incubated for 72 hours, the morphology of neuroretina explants exposed to non-toxic perfluoro-n-octane samples and incubated for 72 hours and the morphology of neuroretina explants exposed to toxic perfluoro-n-octane samples and incubated for 72 hours.

## EXAMPLES

Next, the invention is illustrated by means of assays performed by the inventors that demonstrate the effectiveness of the described method for evaluating the cytotoxicity of chemical volatile compounds particularly of those comprised in healthcare products, on ARPE-19, L929 and neuroretina explant cell cultures.

Example 1. Evaluation of the proliferation of a retinal pigment epithelium cell culture (ARPE-19 cell line) treated with toxic and non-toxic perfluoro-n-octane samples.

The cell line was obtained from the American Tissue Culture Collection (ATCC). After unfreezing them, the cells were cultured in culture medium [(Dulbecco's Modified Eagle Medium and F12 Nutrient Mixture (Ham)] supplemented with 10% of fetal bovine serum and 1% of antibiotic and antifungal (100 U/ml of Penicillin, 100 μg/ml of Streptomycin and 0.25 μg/ml of Amphotericin B). The cell culture was kept in 75 $cm^2$ bottles under standard culture conditions of 37°C and 5% $CO_2$, renewing the culture medium every 2 days and subculturing the cells when they reached a confluence of 80%-90%. When the cells reached said confluence, they were trypsinized and counted using trypan blue. Next, they were sown on a 96-well plate at an optimal density of $1 \times 10^4$ cells/well and incubated for 7 days using complete medium under standard conditions of 37°C and 5% $CO_2$, renewing the culture medium every two days. Next, the complete culture medium was removed and the cells were incubated using incomplete culture medium (without serum but with 1% antibiotic and antifungal) for 24 hours under standard culture conditions, with the aim of synchronizing the cell cycle. Next, 80 μl of the toxic and non-toxic perfluoro-n-octane samples were deposited on the cell culture in the corresponding cells, to expose the cells directly to the perfluoro-n-octanes. Next, 100 μl of culture medium was deposited on the perfluoro-n-octane samples in the wells with the samples, and 180 μl of culture medium in the unexposed wells (Fig. 1). The cells were incubated for 30 minutes and, once said time had elapsed, the perfluoro-m-octane samples and culture medium were removed from each well. The cells were washed twice with PBS (culture medium can also be used) and incubated again with fresh culture medium for 24 hours under standard conditions. Next, cell growth was determined in each well following the MTT cytotoxicity assay protocol, in accordance with the recommendations of the ISO standard (UNE-EN ISO 10993-5:2009 Biological evaluation of healthcare products, Part 5: *In vitro* cytotoxicity assays; Appendix C: MTT toxicity assay].

1.1. Results

The results showed that the perfluoro-n-octane samples cannot evaporate when culture medium is deposited thereon (figure 2B and C). Once the exposure time had elapsed, the samples were able to be collected for the subsequent analysis thereof (figure 3). The cell culture exposed to the non-toxic sample has a viability greater than 70% when compared to the viability of the unexposed cell culture (100%) [(figure 4 A; first column (cells not exposed to the sample), second and third columns (cells exposed to non-toxic samples)]. Thus, it is confirmed that the perfluoro-n-octane sample does not crush the cell culture (figure 4 A; second and third columns) when 80 $\mu$l of the product is deposited. The cell culture exposed to the toxic samples has a viability lower than 70% when compared to the unexposed cell culture and to the non-toxic samples (figure 4 A; fourth and fifth columns). The analysis of the data also confirms that the average $OD_{570}$ value of the unexposed blank group is $\geq 0.2$ after $\geq 7$ days of culture, and that the difference between the average $OD_{570}$ of the blank groups on the right and on the left does not differ by more than 15% of the average of all the blanks. This confirms that the experiments are compliant with the ISO standards.

Example 2. Evaluation of the proliferation of a fibroblast cell culture (L929 cell line) treated with toxic and non-toxic perfluoro-n-octane samples.

The L929 cell line was obtained from the American Tissue Culture Collection (ATCC). After unfreezing them, the cells were cultured in culture medium [(Eagle's Minimum Essential Medium (EMEM) and (ATCC® 30-2003™)] supplemented with 15% of fetal bovine serum and 1% of antibiotic and antifungal (100 U/ml of Penicillin, 100 $\mu$g/ml of Streptomycin and 0.25 $\mu$g/ml of Amphotericin B). The cell culture was kept in 75 cm$^2$ bottles under standard culture conditions of 37°C and 5% $CO_2$, renewing the culture medium every 2 days and subculturing the cells when they reached a confluence of 80%-90%. When the cells reached said confluence, they were trypsinized and counted using trypan blue. Next, they were sown on a 96-well plate at an optimal density of 1x10$^4$ cells/well and incubated for 1 day with complete medium under standard conditions of 37°C and 5% $CO_2$. Next, the complete culture medium was removed and the cells were incubated using incomplete culture medium (without serum but with 1% antibiotic and antifungal) for 24 hours under standard culture conditions, with the aim of synchronizing the cell cycle. Next, 80 $\mu$l of the toxic and non-toxic perfluoro-n-octane samples were deposited on the cell culture in the corresponding cells, to expose the cells directly to the perfluoro-n-octanes. Next, 100 $\mu$l of culture medium was deposited on the perfluoro-n-octane samples in the wells with the samples, and 180 $\mu$l of culture medium in the unexposed wells. The cells were incubated for 30 minutes and, once said time had elapsed, the perfluoro-m-octane samples and culture medium were removed from each well. The cells were washed twice with PBS (culture medium can also be used) and incubated again with fresh culture medium for 24 hours under standard conditions. Next, cell growth was determined in each well following the MTT cytotoxicity assay protocol, in accordance with the recommendations of the ISO standard (UNE-EN ISO 10993-5:2009 Biological evaluation of healthcare products, Part 5: *In vitro* cytotoxicity assays; Appendix C: MTT toxicity assay].

2.1. Results

The results showed that the perfluoro-n-octane samples cannot evaporate when culture medium is deposited thereon (figure 2B and C). Once the exposure time had elapsed, the samples were able to be collected for the subsequent analysis thereof (figure 3). The cell culture exposed to the non-toxic sample has a viability of more than 70% when compared to the viability of the unexposed cell culture (100%) [(figure 4 B; first column, cells not exposed to the sample), second and third columns (cells exposed to non-toxic samples)]. Thus, it is confirmed that the perfluoro-n-octane sample does not crush the cell culture (figure 4 B; second and third columns) when 80 $\mu$l of the product is deposited. The cell culture exposed to the toxic samples has a viability lower than 70 % when compared to the unexposed cell culture and to the non-toxic samples (figure 4 B; fourth and fifth columns). The data analysis also confirms that the average $OD_{570}$ value of the unexposed blank group is $\geq 0.2$ after $\geq 1$ day of culture, and that the difference between the average $OD_{570}$ of the blank groups on the right and on the left does not differ by more than 15% of the average of all the blanks. This confirms that the experiments are compliant with the ISO standards.

Example 3. Evaluation of the alterations in the structure of the neuroretina explants treated with toxic and non-toxic perfluoro-n-octane samples.

The pig eyeballs were dissected within two hours following enucleation to obtain the neuroretina explants. The explants were placed on a 12-well plate and 500 $\mu$l of the toxic and non-toxic perfluoro-n-octane samples were subsequently deposited thereon, thereby exposing the neuroretinas directly to the healthcare product. 500 $\mu$l of culture medium (Neurobasal A medium supplemented with 10% of fetal bovine serum, 1% of antibiotic and antifungal, 1% of L-Glutamin and 2% B-27) were deposited on the perfluoro-n-octane samples to prevent their evaporation. 1 ml of culture medium was deposited on the neuroretina explants for the group of explants not exposed to perfluoro-n-octane. After 30 minutes, the perfluoro-m-octane samples and culture medium were removed from each well. The neuroretina explants were washed with fresh culture medium and transferred to a 12-well plate with Transwell® membranes. The neuroretina explants were deposited on said Transwell® membranes such that the photoreceptor layer remained in contact with the membrane. Each well was filled with culture medium up to the explants but not so much that they float. The plates with the explants were incubated for 72 hours under standard conditions of 37°C and 5% $CO_2$ renewing the culture medium daily. At the same time (0 hours), one of the explants obtained was fixed and stored for processing and observing the morphological changes in the neuroretina explants without being cultured. After 72 hours of culturing, the neuroretina explants were fixed and stored for processing. The neuroretina explants are processed and stained with toluidine blue. Lastly, they were observed under the microscope to evaluate their morphology. The results are shown in Figure 5.

3.1. Results

The results showed that the perfluoro-n-octane samples are not capable of evaporating when culture medium is deposited on the product (figure 2B and C). Once the exposure time had elapsed, the samples were able to be collected for the subsequent analysis thereof (figure 3). The neuroretina explants show good morphology at zero time (figure 5). The neuroretina explants that were exposed to the non-toxic perfluoro-n-octane samples exhibited a morphology similar to that observed in the case of the explants not exposed to the 72 hours of culturing (figure 5). However, the neuroretina explants exposed to the toxic perfluoro-n-octane samples exhibited a deteriorated morphology after 72 hours of culturing (figure 5). Therefore, it is confirmed that the perfluoro-n-octane samples do not crush the neuroretina explants and the time of exposure of the explants to the perfluoro-n-octane samples can be regulated. The experiments are compliant with the ISO standards.

Thus, taking into account the results obtained in the previously described examples, it can be concluded that:

- The designed method does not permit the perfluoro-n-octane, which is a highly volatile substance, to evaporate.
- The designed method makes it possible to regulate the exposure time of the perfluoro-n-octane to the cell and tissue cultures.
- The designed method is capable of detecting the toxic effect of the perfluoro-n-octane samples on RPE cell cultures and on neuroretina explant cultures.
- The designed method facilitates the collection of perfluoro-n-octane samples after the exposure time for the subsequent analysis thereof.

**Claims**

1. A method for evaluating the cytotoxicity of volatile chemical compounds, which comprises:

    a. Depositing a volatile chemical compound on an *in vitro* cell or tissue culture,
    b. Depositing a non-volatile aqueous medium on the compound deposited in step (a),
    c. Incubating the cell or tissue culture obtained after step (b), and
    d. Evaluating the effect of the presence of the compound deposited on the culture; wherein the deposited compound is water-insoluble and has a higher molecular weight than that of the non-volatile aqueous medium of step (b); and
    wherein the amount of compound deposited in step (a) covers the cultured cell or tissue layer and the amount of non-volatile aqueous medium deposited in step (b) covers the compound layer deposited in step (a).

2. The method, according to claim 1, wherein the deposited compound is a compound used in the preparation of medical devices or healthcare products.

3. The method, according to claim 2, wherein the medical devices are used in ophthalmic surgery.

4. The method, according to any of claims 1 to 3, wherein the volatile compound is a manufactured polymer.

5. The method, according to any of claims 1 to 4, wherein the volatile compound is perfluoro-n-octane.

6. The method, according to any of claims 1 to 5, wherein the cell or tissue culture comprises neuroretina explants and/or retinal pigment epithelium cells.

7. The method, according to claim 6, wherein the neuroretina explants and retinal pigment epithelium cells are of human origin.

8. The method, according to any of claims 1 to 7, wherein the non-volatile aqueous medium of step (b) is a culture medium with an identical composition to that used in the cell or tissue culture of step (a).

9. The method, according to any of claims 1 to 8, wherein the incubation of step (c) is carried out for at least 30 minutes.

10. The method, according to any of claims 1 to 9, wherein the cell proliferation and/or survival or morphology of the tissue is evaluated in step (d).

11. The method, according to any of claims 1 to 10, which further comprises a step (e) wherein the compound deposited in step (a) is recovered.

12. The method, according to any of claims 1 to 11, wherein the method is carried out in a multi-well plate wherein at least two columns of the plate comprise control cell or tissue cultures.

13. The method, according to claim 12, wherein the average optical density value ($OD_{570}$) of the control cell cultures of the two columns is at least 0.2.

14. The method, according to claim 13, wherein the difference between the average $OD_{570}$ value of the control cell cultures of the two columns is equal to or less than 15% of the total average of both columns.

15. The method, according to any of claims 12 to 14, wherein the control cultures comprise cells or tissue not exposed to any compound, cells or tissue exposed to a non-toxic compound with a molecular weight equal to that of the deposited compound under study or cells or tissue exposed to a control toxic compound.

16. The method, according to claim 15, wherein the control toxic compound is selected from the list consisting of: PVC-org. Sn, SPU-ZDEC, SPU-ZBEC, natural latex and polyurethane or phenol.

17. The method, according to any of claims 15 or 16, wherein the cytotoxicity of the cell culture studied is compared to the cytotoxicity of the control cultures that comprise cells not exposed to any compound following the formula:

$$\text{Viab.\%} = \frac{100 * OD570e}{OD570b}$$

OD570e being the average optical density value in the cell culture studied and OD570b being the average optical density value in the control culture that comprises cells not exposed to any compound.

**Patentansprüche**

1. Verfahren zum Beurteilen der Zytotoxizität von flüchtigen chemischen Verbindungen, das umfasst:

   a. Abscheiden einer flüchtigen chemischen Verbindung auf eine *in vitro*-Zell- oder Gewebekultur,
   b. Abscheiden eines nicht flüchtigen wässrigen Mediums auf die in Schritt (a) abgeschiedene Verbindung,
   c. Inkubieren der nach Schritt (b) erhaltenen Zell- oder Gewebekultur und
   d. Beurteilen der Auswirkung auf das Vorhandensein der auf die Kultur abgeschiedenen Verbindung; wobei die abgeschiedene Verbindung wasserunlöslich ist und ein höheres Molekulargewicht aufweist als das nicht flüchtige wässrige Medium von Schritt (b); und

   wobei die Menge der in Schritt (a) abgeschiedenen Menge die kultivierte Zell- oder Gewebeschicht bedeckt und die Menge an in Schritt (b) abgeschiedenem nicht flüchtigem wässrigen Medium die in Schritt (a) abgeschiedene Verbindungsschicht bedeckt.

2. Verfahren nach Anspruch 1, wobei die abgeschiedene Verbindung eine Verbindung ist, die bei der Herstellung von medizinischen Vorrichtungen oder Gesundheitspflegeprodukten verwendet wird.

3. Verfahren nach Anspruch 2, wobei die medizinischen Vorrichtungen in der Augenchirurgie verwendet werden.

4. Verfahren nach einem der Ansprüche 1 bis 3, wobei die flüchtige Verbindung ein gefertigtes Polymer ist.

5. Verfahren nach einem der Ansprüche 1 bis 4, wobei die flüchtige Verbindung Perfluor-n-Octan ist.

6. Verfahren nach einem der Ansprüche 1 bis 5, wobei die Zell- oder Gewebekultur Neuroretina-Explantate und/oder retinale Pigmentepithelzellen umfasst.

7. Verfahren nach Anspruch 6, wobei die Neuroretina-Explantate und retinalen Pigmentepithelzellen menschlichen Ursprungs sind.

8. Verfahren nach einem der Ansprüche 1 bis 7, wobei das nicht flüchtige wässrige Medium von Schritt (b) ein Kulturmedium mit einer identischen Zusammensetzung wie desjenigen ist, das in der Zell- oder Gewebekultur von Schritt (a) verwendet wird.

9. Verfahren nach einem der Ansprüche 1 bis 8, wobei die Inkubation von Schritt (c) mindestens 30 Minuten lang ausgeführt wird.

10. Verfahren nach einem der Ansprüche 1 bis 9, wobei die Zellproliferation und/oder das Überleben oder die Morphologie des Gewebes in Schritt (d) beurteilt wird.

11. Verfahren nach einem der Ansprüche 1 bis 10, das ferner einen Schritt (e) umfasst, wobei die Verbindung, die in Schritt (a) abgeschieden wird, wiederhergestellt wird.

12. Verfahren nach einem der Ansprüche 1 bis 11, wobei das Verfahren in einer Multi-Well-Platte ausgeführt wird, wobei mindestens zwei Säulen der Platte Kontrollzell- oder -gewebekulturen umfassen.

13. Verfahren nach Anspruch 12, wobei der durchschnittliche optische Dichtewert ($OD_{570}$) der Kontrollzellkulturen der zwei Säulen mindestens 0,2 beträgt.

14. Verfahren nach Anspruch 13, wobei der Unterschied zwischen dem $OD_{570}$-Wert der Kontrollzellkulturen der zwei Säulen gleich oder kleiner ist als 15% des Gesamtdurchschnitts beider Säulen.

15. Verfahren nach einem der Ansprüche 12 bis 14, wobei die Kontrollkulturen Zellen oder Gewebe, die keiner Verbindung ausgesetzt werden, Zellen, oder Gewebe, die einer nicht toxischen Verbindung mit einem Molekulargewicht gleich demjenigen der abgeschiedenen Verbindung der Untersuchung ausgesetzt werden oder Zellen oder Gewebe, die einer toxischen Kontrollverbindung ausgesetzt werden, umfassen.

16. Verfahren nach Anspruch 15, wobei die toxische Kontrollverbindung ausgewählt ist aus der Liste, bestehend aus: PVC-org. Sn, SPU-ZDEC, SPU-ZBEC, natürlichem Latex und Polyurethan oder Phenol.

17. Verfahren nach einem der Ansprüche 15 oder 16, wobei die Zytotoxizität der untersuchten Zellkultur mit der Zytotoxizität der Kontrollkulturen verglichen wird, die Zellen umfassen, die keiner Verbindung gemäß der folgenden Formel ausgesetzt werden:

$$\text{Lebensfäh. \%} = \frac{100 * OD570e}{OD570b}$$

wobei OD570e der durchschnittliche optische Dichtewert in der untersuchten Zellkultur ist und OD570b der durchschnittliche optische Dichtewert in der Kontrollkultur ist, die Zellen umfasst, die keiner Verbindung ausgesetzt werden.

**Revendications**

1. Procédé pour évaluer la cytotoxicité de composés chimiques volatils, qui comprend :

a. Dépôt d'un composé chimique volatil sur une cellule cellulaire ou tissulaire *in vitro,*
b. Dépôt d'un milieu aqueux non volatil sur le composé déposé à l'étape (a),
c. Incubation de la culture cellulaire ou tissulaire obtenue après l'étape (b), et
d. Évaluation de l'effet de la présence du composé déposé sur la culture ; dans lequel le composé déposé est insoluble dans l'eau et a un poids moléculaire supérieur à celui du milieu aqueux non volatil de l'étape (b) ; et

dans lequel la quantité de composé déposée à l'étape (a) recouvre la couche cellulaire ou tissulaire cultivée et la quantité de milieu aqueux non volatil déposée à l'étape (b) recouvre la couche de composé déposée à l'étape (a).

2. Procédé, selon la revendication 1, dans lequel le composé déposé est un composé utilisé dans la préparation de dispositifs médicaux ou de produits de santé.

3. Procédé, selon la revendication 2, dans lequel les dispositifs médicaux sont utilisés en chirurgie ophtalmique.

4. Procédé, selon l'une quelconque des revendications 1 à 3, dans lequel le composé volatil est un polymère fabriqué.

5. Procédé, selon l'une quelconque des revendications 1 à 4, dans lequel le composé volatil est le perfluoro-n-octane.

6. Procédé, selon l'une quelconque des revendications 1 à 5, dans lequel la culture cellulaire ou tissulaire comprend des explants de neurorétine et/ou des cellules d'épithélium pigmentaire rétinien.

7. Procédé, selon la revendication 6, dans lequel les explants de neurorétine et les cellules d'épithélium pigmentaire rétinien sont d'origine humaine.

8. Procédé, selon l'une quelconque des revendications 1 à 7, dans lequel le milieu aqueux non volatil de l'étape (b) est un milieu de culture avec une composition identique à celle utilisée dans la culture cellulaire ou tissulaire de l'étape (a).

9. Procédé, selon l'une quelconque des revendications 1 à 8, dans lequel l'incubation de l'étape (c) est effectuée pendant au moins 30 minutes.

10. Procédé, selon l'une quelconque des revendications 1 à 9, dans lequel la prolifération cellulaire et/ou la survie ou la morphologie du tissu est évaluée à l'étape (d).

11. Procédé, selon l'une quelconque des revendications 1 à 10, qui comprend en outre une étape (e) dans laquelle le composé déposé à l'étape (a) est récupéré.

12. Procédé, selon l'une quelconque des revendications 1 à 11, dans lequel le procédé est effectué dans une plaque à puits multiples dans laquelle au moins deux colonnes de la plaque comprennent des cultures cellulaires ou tissulaires témoins.

13. Procédé, selon la revendication 12, dans lequel la valeur de densité optique moyenne $(DO_{570})$ des cultures cellulaires témoins des deux colonnes est d'au moins 0,2.

14. Procédé, selon la revendication 13, dans lequel la différence entre la valeur $DO_{570}$ moyenne des cultures cellulaires témoins des deux colonnes est égale ou inférieure à 15 % de la moyenne totale des deux colonnes.

15. Procédé, selon l'une quelconque des revendications 12 à 14, dans lequel les cultures témoins comprennent des cellules ou du tissu non exposés à un quelconque composé, des cellules ou du tissu exposés à un composé non toxique avec un poids moléculaire égal à celui du composé déposé à l'étude ou des cellules ou du tissu exposés à un composé toxique témoin.

16. Procédé, selon la revendication 15, dans lequel le composé toxique témoin est choisi dans la liste constituée de : PVC-org. Sn, SPU-ZDEC, SPU-ZBEC, latex naturel et polyuréthane ou phénol.

17. Procédé, selon l'une quelconque des revendications 15 ou 16, dans lequel la cytotoxicité de la culture cellulaire étudiée est comparée à la cytotoxicité des cultures témoins qui comprennent des cellules non exposées à un quelconque composé suivant la formule :

$$\text{Viab.\%} = \frac{100 * DO570e}{DO570b}$$

DO570e étant la valeur de densité optique moyenne dans la culture cellulaire étudiée et DO570b étant la valeur de densité optique moyenne dans la culture témoin qui comprend des cellules non exposées à un quelconque composé.

**Fig. 1**

Biological, physical and chemical analysis

**Fig. 2**

Fig. 3

## Fig. 4 (A)

## Fig. 4 (B)

**Fig. 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LI, W. ; ZHOU, J. ; XU, Y.** *Biomedical Reports,* 2015, vol. 3 (5), 617-620
- **FRICKER, S.** *Toxicology in Vitro,* 1994, vol. 8 (4), 879-881
- **ZHONGGUO YI ; LIAO QI ; XIE ZA ZHI.** *In Vitro Cytotoxicity Study of Nickel Ion,* March 2015, vol. 39 (3), 212-5
- **YANG et al.** *Toxicology In Vitro,* 2002, vol. 16 (1), 33-39